# EUROPEAN PATENT APPLICATION

(11) **EP 4 483 795 A1**
(43) Date of publication of application: **01.01.2025**
(21) Application number: 23181553.1
(22) Date of filing: 26.06.2023
(51) Int. Cl.: A61B 5/024, A61B 5/145, A61B 5/1455, A61B 5/1464, A61B 5/288, A61B 5/344, A61B 5/00

(54) **AN INTRA-PARTUM FETAL MONITORING SYSTEM**

(71) Applicant: University College Cork, National University Of Ireland, Cork (IE)
(72) Inventor: BURKE, Ray, Cork (IE); MCCARTHY, Fergus, Cork (IE)
(74) Representative: FRKelly

(57) **Abstract**

The present invention provides an intra-partum fetal monitoring system, and in particular a real time fetal heartrate and maternal contraction monitoring system for detection and prevention of perinatal hypoxia, the device including an optical sensor operable monitor fetal heartrate and a motion based contraction sensor operable to monitor maternal contraction, in addition to a processor operable to convert real time data from the heartrate sensor and contraction sensor into cross correlated real time data regarding intra-partum fetal wellbeing.

## Description

### Field of the Invention

This invention relates to an intra-partum fetal monitoring system, and in particular a real time fetal heartrate monitoring in combination with maternal contraction monitoring, with optional fetal pH, lactate and physiological monitoring for monitoring intra-partum fetal and maternal wellbeing.

### Background of the Invention

The monitoring of fetal vital signs intra-partum (in labour) is currently routinely done using cardiotocography (CTG) heart rate monitor using the Doppler ultrasound principle to measure the mechanical motion of the babies heart (over the mothers motions) and in addition and when in crisis with a very invasive fetal scalp blood sampling needle and blood gas procedure.

While perinatal hypoxia or asphyxia during labour is the key risk to the fetus there are many parameters (some not yet clinically investigated) related to and potentially contributing to the gross fetal hypoxia. Hypoxia is the partial or complete lack of oxygen to neonates at the time of labour and delivery. This devastating complication of childbirth is associated with up to 60% of babies dying and represents a significant unmet clinical need in maternal and infant care. Intra-partum hypoxia can lead to several serious medical conditions affecting the neurological system, resulting in cases such as Hypoxic Ischemic Encephalopathy (HIE) and other brain injuries that may lead to paralysis and severe brain damage causing debilitating cognitive delays, developmental problems, and motor impairments.

All babies born by vaginal delivery are potentially at risk of hypoxic injury. Suspected hypoxic injury assessed by current state of the art heart rate monitoring instruments, has been a long-standing and common problem managed in labour wards by obstetricians and midwives globally.

Current methods of fetal monitoring during labour include cardiotocography (CTG; fetal heart rate monitoring), the current gold standard and Fetal Blood Sampling (FBS). These technologies are limited by technical challenges such as the necessity for advanced cervical dilation, requirement for regional analgesia or volume of blood required to ascertain fetal pH. Use of computerised interpretation of the ultrasound based CTG in women who have continuous electronic fetal monitoring in labour has also been shown to not improve clinical outcomes for mothers or babies (Brocklehurst et al, Lancet 2017). As a result, inadvertent caesarean sections are performed due to false concerns around CTG readings. Furthermore, misinterpretation, or inability to interpret the CTG, resulting in hypoxic brain injury is a cause of litigation exceeding 1 billion GBP in the UK annually. The CTG data is also frequently very difficult to accurately read, and an aggravating factor is that the sensors utilised are not robust and frequently stop working or need to be adjusted on the mother leading to gaps in the data recording with associated clinical risk due to lack of monitoring. One other key issue with CTG arises from the use of transcutaneous maternal monitoring of the fetal heartrate which is frequently synchronised to the maternal heart rate giving incorrect and misleading data. Under normal conditions the fetal heartrate is in the region of 140bpm while the maternal heartrate is in the region of 70bpm. However during labour the maternal heartrate is often significantly elevated due to factors such as physical stress, the administration of an epidural, etc. and as a result the fetal and maternal heartrates can overlap or merge making it extremely difficult to distinguish the two with CTG, thus resulting in an absence of useful data for the clinician.

There is therefore a need for an improved means of detecting and therefore preventing fetal hypoxic brain injury. Accurate detection of fetal hypoxia will ensure that caesarean sections are performed only when absolutely required. This contrasts with the current situation where reliance on fetal heart rate monitoring which has poor sensitivity and specificity for fetal hypoxia results in high levels of Caesarean section incorrectly performed for suspected fetal distress.

Given that 75% of labour cases occur gradually, preventative and continuous monitoring is highly desirable at this point in the patient pathway and can result in positive patient outcomes, an improved level of maternity care and a long-term reduction in patient care costs in both mother and child.

It is therefore an object of the present invention to provide a fetal monitoring system which improves the degree of accuracy and reliability of fetal monitoring when compared to the current gold standard.

### Summary of the invention

According to a first aspect of the present invention there is provided an intra-partum fetal monitoring system comprising a probe adapted for in-utero contact with vascularised tissue on the head of a fetus and operable to monitor fetal heartrate and/or fetal blood oxygen levels; a contraction sensor adapted to monitor maternal contractions; and a processor operable to convert real time data from the probe and real time data from the contraction sensor into correlated real time data regarding one or more biological markers to enable an assessment of intra-partum fetal wellbeing.

Preferably, the probe comprises at least one optical sensor operable to emit light at a wavelength of between 500nm-950nm onto the vascularised tissue and to detect the reflected light.

Preferably, the optical sensor is operable to simultaneously and/or sequentially emit and detect light at different wavelengths and/or wavelength bands.

Preferably, the optical sensor comprises at least one multispectral light source.

Preferably, the optical sensor is adapted to achieve an optical depth of penetration into the vascularised tissue of less than 5mm, more preferably less than 3mm and most preferably less than 2mm.

Preferably, the contraction sensor comprises a three dimensional motion sensor and/or a vibration sensor and/or an electromyography sensor.

Preferably, the contraction sensor comprises a heartrate sensor operable to measure maternal heartrate.

Preferably, the heartrate sensor comprises at least one optical sensor operable to emit light onto maternal tissue and to detect the reflected light.

Preferably, the contraction sensor comprises at least one strain gauge.

Preferably, the contraction sensor comprises a wearable band incorporating the at least one strain gauge.

Preferably, the probe and/or the contraction sensor comprises at least one optical emitter and/or at least one optical detector.

Preferably, the probe and/or the contraction sensor comprises multiple optical emitters adapted to emit light of different wavelengths.

Preferably, at least one of the optical detectors comprises a spectrometer.

Preferably, the probe and/or the contraction sensor comprises a temperature sensor and/or an impedametric sensor.

Preferably, the probe comprises a discrete pH sensor.

Preferably, the probe and/or the contraction sensor comprises a discrete power supply.

Preferably, the processor is operable to convert real time spectroscopy data from the probe into measurements of one or more of fetal blood lactate level, blood pH level, blood oxygen saturation, blood carbon dioxide level, body temperature, hydration and heart rate.

Preferably, the probe is operable to monitor oxygen saturation.

Preferably, the probe comprises two or more electrodes operable to convey an electrical signal through the vascularised tissue to enable measurement of hydration and/or pH.

Preferably, the probe and/or the contraction sensor comprises an electroencephalogram sensor.

Preferably, the monitoring system comprises a vacuum cup for securing the probe to the vascularised tissue.

Preferably, the monitoring system comprises a displacement sensor operable to monitor the distance between the probe and a reference location.

Preferably, the displacement sensor is operable to monitor the distance between the probe and the contraction sensor.

Preferably, the monitoring system comprises a remote processing unit containing the processor.

Preferably, the monitoring system comprises a tether connecting the probe and the processing unit.

Preferably, the tether defines an electrical, optical and/or fluid communication path between the probe and the processing unit.

According to a second aspect of the present invention there is provided a method of in-utero monitoring of fetal levels of selected biomarkers, the method comprising the steps of monitoring fetal heartrate and/or fetal blood oxygen levels; monitoring maternal contractions; and converting real time data from the fetal heartrate and/or fetal blood oxygen levels and real time date from the maternal contractions into correlated data regarding intra-partum fetal wellbeing.

Preferably, the method comprises emitting light onto the vascularised tissue and detecting the reflected light in order to monitor the fetal heartrate and/or fetal blood oxygen levels.

Preferably, the method comprises converting real time spectroscopy data from the reflected light into measurements of one or more of blood lactate level, blood pH level, blood oxygen saturation, blood carbon dioxide level, body temperature, hydration and heart rate.

Preferably, the method comprises measuring three dimensional movements of the maternal abdomen in order to detect and/or monitor maternal contractions.

Preferably, the method comprises measuring electrical activity in maternal muscle in order to detect and/or monitor maternal contractions.

Preferably, the method comprises monitoring maternal heartrate and converting the real time data into correlated data regarding intra-partum fetal wellbeing.

### Brief description of the invention

The present invention will now be described with reference to the accompanying drawings, in which:
Figure 1 illustrates a perspective view of a probe forming part of a fetal monitoring system according to the present invention;
Figure 2 illustrates a sectioned perspective of the probe of Figure 1;
Figure 3 illustrates a side elevation of the probe shown in Figure 1;
Figure 4 illustrates a sectioned side elevation of the probe shown in Figure 1;
Figure 5 illustrates a bottom plan view of the probe of Figures 1 to 4;
Figure 6 illustrates an exploded perspective view from above of the probe shown in Figure 1;
Figure 7 illustrates an exploded perspective view from below of the probe shown in Figure 1;
Figure 8 illustrates a schematic representation of a contraction sensor forming part of a fetal monitoring system according to the present invention, including a band for securing the sensor to the maternal abdomen;
Figure 9 illustrates a schematic representation of the contraction sensor of Figure 8 in isolation;
Figure 10 illustrates a perspective view of an alternative embodiment of a probe forming part of a fetal monitoring system according to the present invention located on the finger of a medical professional; and
Figure 11 illustrates the probe of Figure 9 from a position above the hand of the medical professional.

### Detailed description of the drawings

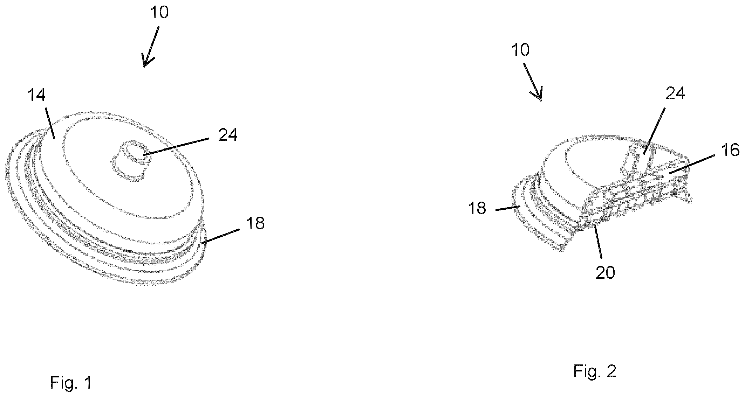

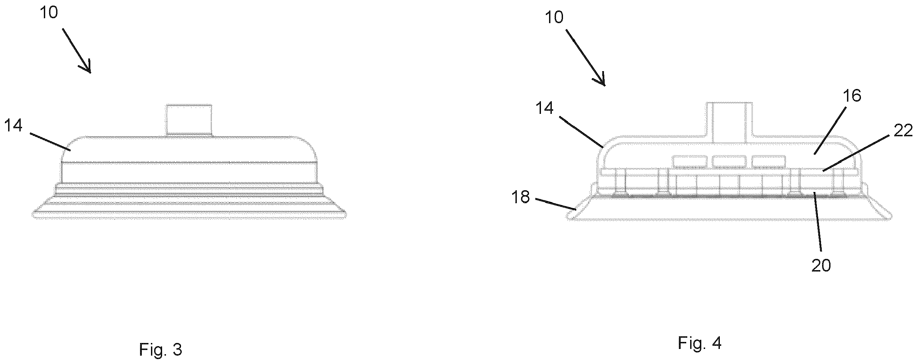

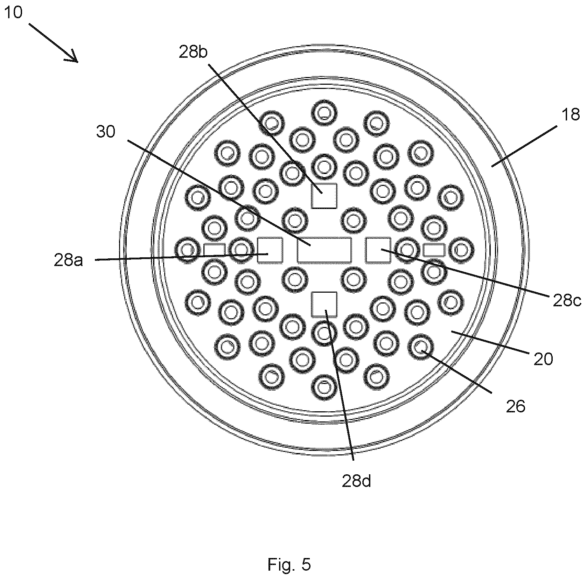

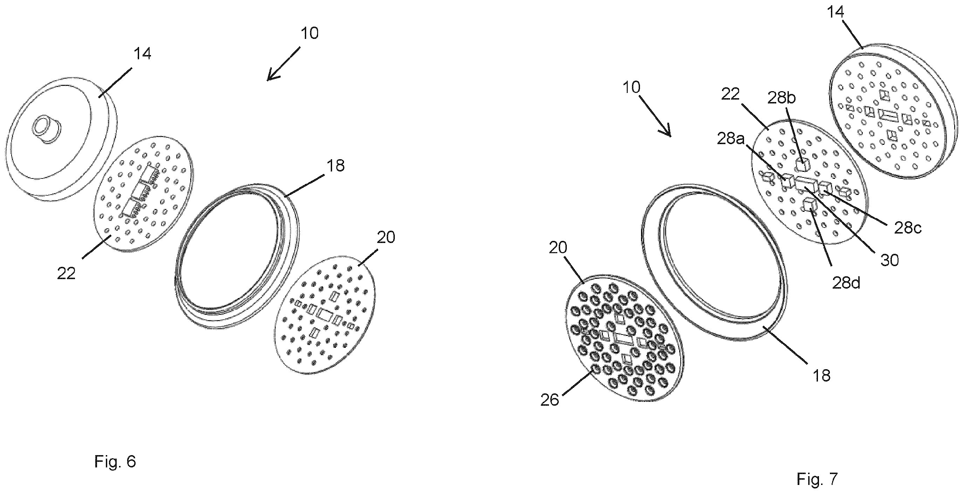

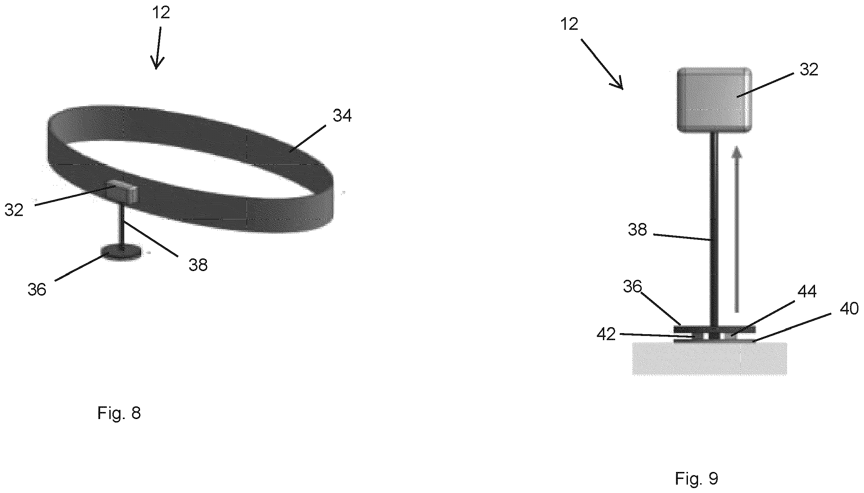

Referring now to Figures 1 to 9 of the accompanying drawings there is illustrates a fetal probe forming part of a fetal monitoring system according to the invention, the probe generally indicated as 10, for use in real time monitoring of fetal bio-signs, in particular pulsatile heartrate but optionally also in a multimodal manner to measure/monitor blood oxygen concentration, fetal temperature and hydration, blood lactate and pH, all in order to monitor the health of the fetus in particular in relation to hypoxia during labour. The monitoring system additionally comprises a contraction sensor 12 an embodiment of which is shown in Figures 8 and 9 and which in use monitors maternal contractions through one or more modes of operation and the data from which is combined with data from the probe 12 in order to accurately monitor intra-partum fetal wellbeing.

As explained in detail hereinafter, the probe 10 of the monitoring system preferably operates using reflectance based sensing and is placed on the fetal scalp during labour at the time of routine vaginal examination by a health professional to provide a real-time readout of at least fetal heartrate, but optionally the above additional bio-signs. The probe 10 is also capable of multimodal operation in order to additionally monitor temperature, heat flow, pCOz (partial pressure of carbon dioxide), and pOz (partial pressure of oxygen) which data can be used to augment the measurement of the principle components or key measured parameters of heartrate, oxygen concentration, lactate and pH to facilitate improved data processing by a novel algorithm executable on a processor (not shown) forming part of the monitoring system to thereby provide supporting data to the clinicians. The monitoring system most notably provides a clear and accurate fetal heartrate from which wellbeing can be determined, whether by the clinician interpreting the heartrate and related intra-partum factors, or optionally by the processor and associated algorithm, or a combination of both. By utilising the probe 10 directly on the fetal scalp a direct heartrate is acquired and so can be separately and distinctly displayed relative to the maternal heartrate, providing clear data to the clinician throughout labour.

The probe 10 of the monitoring system accesses the capillary bed non-invasively via light that is emitted by the probe 10, reflected off the fetal scalp, and collected/detected by the probe in order to allow the processor (not shown) to utilise the reflected light to measure heartrate, known as reflectance based heartrate monitoring. The probe 10 preferably comprises a housing 14 which is shaped and dimensioned to be attached to the fetal scalp in the fontanelle region without piercing the epidermis of the fetus. The housing 14 comprises a plenum chamber 16 circumscribing one end of which is a conical or flared skirt 18 formed of biocompatible and resiliently deformable material to facilitate conforming contact against the fetal head in use. The plenum chamber 16 comprises a perforated or gas permeable end wall 20 adjacent the skirt 18, with a sensor substrate such as a printed circuit board 22 captured within the plenum chamber 16 and carrying various sensors, as, detailed hereinafter, which can communicate with the fetal scalp in order to measure heartrate and other bio-markers or parameters.

The housing 14 further comprises a connector 24 to which in use is connected a tether (not shown), to enable certain functional, for example providing power to the probe 10, providing negative pressure to the probe to facilitate suction to the scalp, and to facilitate data communication communicate with one or more external systems (not shown) for data processing, light delivery and detection, etc., but also to provide an externally accessible physical connection to allow the probe 10 to be quickly and easily retrieved if required. However, the probe 10 of the monitoring system of the invention may also be provided in an untethered form between which communication may be achieved wirelessly.

The tether (not shown) defines a communication path to the housing 14, and for example may define a vacuum path which terminates at a plurality of openings 26 in the end wall 20, allowing a vacuum pump (not shown) or the like to communicate with the interior space defined between the skirt 18 and the fetal scalp. The plenum chamber 16 can then be used to apply a vacuum within this space to adhered the housing 14 to the scalp, preferably with the end wall 20 drawn downwardly into close proximity or contact with the fetal scalp in order to improve the efficacy of the one or more sensors described in detail hereinafter. One or more of said sensors may be used to monitor for contact of the end wall 20 with the scalp in order to control the level of vacuum applied in order to securely but safely maintain adherence of the housing 14 to the scalp. The housing 14 then provides a protective enclosure about the vascularised tissue of the head of the fetus during operation of the fetal monitoring system.

In addition to the above functionality the vacuum functionality of the probe 10 may be used to affect a flushing function in order to clear the space defined between the housing 14 and the scalp before the housing 14 is adhered in place. This may be achieved by using a reduced negative pressure sufficient to remove the presence of any interference such as biological fluids or other matter which might adversely affect the operation or accuracy of the sensors. Additionally or alternatively a flushing liquid or gas may be pumped down the tether (not shown) into the space between the housing 14 and scalp, and subsequently withdrawn through the tether in order to remove the flushing liquid and entrained contaminants. Once the flushing action has been completed the negative pressure applied through the tether may be suitably increased to affect adherence of the housing 14 to the scalp. It will be appreciated that the negative pressure may be applied via any suitable means (not shown), and in the present embodiment from a location remote from the probe 10. A biocompatible adhesive (not shown) may also be provided on the interior surface of the skirt 18 in order to further improve adhesion to the fetal scalp.

In order to achieve reflectance heartrate monitoring the probe 10 comprises a discrete optical sensor comprising at least one emitter, and in the preferred embodiment comprising a first emitter 28a, a second emitter 28b, a third emitter 28c and a fourth emitter 28d along with a corresponding optical detector 30, all of which are mounted on the printed circuit board 22 to be in optical communication with the fetal scalp in use. In the embodiment illustrated the end wall 20 is provided with apertures through which light can pass to or from the emitters 28a, 28b, 28c, 28d and the detector 30, although it will be appreciated that the end wall 20 may be solid but provided with optically transparent regions or the like to permit the transmission of light therethrough. The emitters 28a, 28b, 28c, 28d are operable to emit light at a particular wavelength, preferably in the infrared and red bands of the electromagnetic spectrum, and may comprise any suitable light source such as an LED, SLED or laser. The emitters 28a, 28b, 28c, 28d preferably emit light at different wavelengths, and may be operated sequentially or simultaneously. For measuring heartrate the most effective wavelength is in the region of 530nm, but it is preferable that a number of different wavelengths are utilised in order to allow different parameters, such as SpOz to also be measured. While four emitters are employed in the preferred embodiment it should be understood that a greater or lesser number may be employed. The four emitters 28a, 28b, 28c, 28d preferably emit light at 530nm, 600nm and 800nm, with one of the emitters 28a, 28b, 28c, 28d being used to emit light at a certain wavelength for use as a baseline measure. It is also envisaged that a single emitter could be employed that is operable to emit light of different wavelengths, for example a supercontinuum source. Such a single emitter may be switched between the different wavelengths at intervals in order to monitor each. It is further envisaged that light may be transmitted through the tether (not shown) from an external light source and the reflected light returned back through the tether to an external detector. This external detector may also comprise a spectrometer (not shown) to allow complex analysis of the reflected light as detailed hereinafter, for example in order to allow measurement of SpOz levels, pH, etc.

The tether (not shown) can transfer the reflected light from the fetal scalp to this spectrometer (not shown) or detectors located in a hand held unit (not shown) or a typical medical device console unit (not shown). The reflected light can be processed by the spectrometer to provide the corresponding blood lactate and/or pH level, being key biomarkers, to reflect fetal systemic acid base balance and predict adverse neonatal outcomes. The processor running the algorithm is preferably adapted to analyse the spectral data and the processor may be part of the above mentioned hand held unit or medical device console, but it is also envisaged that the processor may be housed locally at the housing 14.

The emitters 28a, 28b, 28c, 28d preferably use multiple wavelengths, for example four to six different wavelengths. In an exemplary embodiment one of the emitters 28a, 28b, 28c, 28d emits light at 530nm, on at 660nm, one at 805nm and another at 900nm. The emitters are preferably spaced from one another, and the detector 30 is operable to measure light reflected off the fetal scalp from the emitters 28a, 28b, 28c, 28d. The signals from the detector 30 are transmitted to the processor (not shown) in order to allow the fetal heartrate to be determined via conventional reflectance based processing techniques. The signals are taken in a time series and cross correlated in an algorithm running on the processor (not shown) to generate an accurate heartrate signal that is suitable for measuring the fetal heartrate trend in addition to any rapid changes which may be indicative of fetal distress. The heart rate may be cross-correlated with the timings for the contractions as monitored by the contraction sensor 12 described in detail hereinafter. The signals from the sensors may be presented simultaneously on any suitable display(s) whether provided as an integral part of the monitoring system or otherwise, allowing constant monitoring by the clinical personnel.

The discrete optical sensor comprising the emitters 28a, 28b, 28c, 28d and detector 30 is particularly configured to perform real time measurement of pulsatile heartrate based on the volumetric change of blood in the measurement area using light at 530nm, but may also be operable to emit light of one or more wavelengths suitable to enable real time measurement of SpOz, also known as reflectance pulse oximetry. As noted above the monitoring system may also comprise a spectrometer (not shown) which may be located remotely of the housing 14, or may be a micro-spectrometer located directly in or on the housing 14. The optical sensor may be operated sequentially or simultaneously with spectrometer based sensing. The data obtained regarding heartrate and/or SpOz can then be utilised by the data processing algorithm employed by the monitoring system to improve the accuracy of the real time clinical diagnosis.

The optical sensor is therefore capable of measuring pulsatile heartrate of the fetus directly, continuous SpO₂ and SpO₂ trends, temperature and heat flow. The SpOz or tissue oxygen saturation (StOz) measurement will preferably but not essentially use the same emitters 28a, 28b, 28c, 28d and detector 30, but as noted above these may be supplemented with a local or remote fibre coupled spectrometer (not shown) to measure SpOz. The optical sensor will in use be calibrated to measure the lower saturation levels of SpOz in the fetus, typically from 30% to 60%. For pH/lactate measurement the light from the emitters 28a, 28b, 28c, 28d is preferably at wavelengths from 1350nm to 2500nm. While the absolute value of SpOz in isolation may not be useful for diagnosis, when combined with the other data and a trend of SpOz over time this data can be usefully processed by the algorithm for the detection of fetal hypoxia. All of the light sources utilised in the monitoring system preferably operate at low power levels for patient and eye safety. The sources may be pulsed light sources to support power management of as part of a data processing strategy.

In addition to the optical sensor the monitoring system, most preferably the probe 10, may be provided with additional sensors (not shown) operable to measure bio-markers or other parameters such a fetal hydration, EEG and EIS. The probe 10 may therefore comprises a temperature sensor (not shown) which may be a thermistor or any other suitable component or assembly of components and which may be arranged to be in close or direct contact, in use, with the fetal scalp and is therefore preferably mounted on the printed circuit board 22 in a suitable location. The temperature sensor may additionally or alternatively be configured for monitoring heat flow (area temperature measurement). As an alternative to a thermistor the temperature sensor may comprise for example a Mid Infrared (MIR) non-contact sensor (not shown) to measure temperature such as the MLX90614 which can be used to measure temperature to an accuracy of +- 0.1°C. This type of sensor requires a stand-off height from the fetal scalp and will measure the temperature over an area from the MIR radiation emitted from the fetus. An accurate measurement of temperature for the fetus is an important diagnostic parameter which is not currently measured in real time but when combined with heartrate may enhance the real time clinical assessment of fetal well-being. The fetal intra-partum fetal temperature can be monitored over time for changes and could indicate a fetal and/or maternal infection. High temperatures (pyrexia) during labour may also aggravate an existing hypoxia due to increased tissue oxygen consumption and a shift of the oxygen dissociation curve to the right. Furthermore, the risk of cerebral palsy increases in children born by mothers with clinical signs of chorioamnionitis and fever.

The monitoring system, and most likely the probe 10, preferably further comprise an impedance/capacitance/resistance sensor (not shown) which may for example comprise a pair of discrete electrodes (not shown) of metal or other suitable material, which may be insulated or not, and which may again be arranged to be in close or direct contact, in use, with the fetal scalp. This sensor is preferably operable to use the electrodes (not shown) to detect tissue water content or hydration in the fetal scalp by means of electrochemical impedance spectroscopy (EIS) but is not limited to measuring this parameter. The electrodes may be electrically insulated or contact metal electrodes. The electrodes may also be utilized to detect biomarkers and/or tissue types and to determine correct placement of the housing 14 to ensure suitable and accurate placement of the various sensors therein. In use a low frequency AC current, in the order of micro-amps, is applied across the electrodes, and the frequency is changed overtime, to measure impedance at different frequencies and to therefore measure certain parameters, most notably hydration. It is also envisaged that the impedance/capacitance/resistance sensor may use a remote impedance spectrometer or discreet electronics designed and tuned to detect hydration.

The monitoring system, and most likely the probe 10, may additionally comprise an EEG and/or an EIS sensor (not shown). For example a subset 4-8 EEG electrode could be located on the printed circuit board 11 to be placed on the head of the fetus to measure fetal distress by analysis of the multichannel EEG signals. The EIS sensor (not shown) may utilise the electrodes from the above mentioned hydration sensor and a remote impedance spectrometer (not shown) to analyse the impedance spectra in the voltage (electric field) and frequency domain. These spectra will indicate certain biomarkers like pH as they are charged particles and will enable determination of different tissue types due to their water content and polarity.

Referring now to Figures 8 and 9 the contraction sensor 12 forming part of the monitoring system of the invention is illustrated. In the preferred embodiment the contraction sensor 12 is adapted for location anteriorly below the maternal naval and is preferably arranged to detect labour contractions via muscle contractions, and optionally via motion and/or vibrations and EMG, and to transmit data on the monitored parameters to the processor (not shown) to be used in assessing intra-partum fetal wellbeing. The contraction sensor 12 comprises a main body 32 which houses a three dimensional motion sensor (not shown) which may for example comprise a magnetometer, accelerometer or other piezo-electric based three dimensional motion/vibration sensor and which is preferably operable to measure movement and vibration on three axes to measure physical movements associated with contractions. These contractions will be a complex series of movements that will have a higher and a lower frequency component arising from large scale maternal movements and more localised high frequency movement and/or vibrations arising from contractions and from which the processor algorithm will filter and extract the accurate contraction duration and assist in predicting contractions. The contraction sensor 12 preferably additionally comprises at least one strain gauge, for example a resistance based stretchable element secured around at least a portion of the material abdomen which will vary in electrical resistance in response to applied force/stretching, allowing measurement of contraction movements.. The contraction sensor 12 may also measure and use maternal EMG at and below the naval to determine contractions and their onset and duration. EMG data is useful in detecting the onset of a contraction, as early electrical activity in the muscle in the pre-contraction phase can be used to indicate the onset of a contraction.

The contraction sensor 12 may be secured in position by any suitable means, and in the embodiment illustrated comprises a band 34 adapted to be worn around the abdomen to secure the main body 32 in position. The band 34 preferably incorporates the one or more strain gauges (not shown) which may then be connected to the main body 32 to allow signal capture and transmission to the processor. It will however be understood that the one or more strain gauges may be provided separately to the band 34, and may communicate data to the processor independently. The data from the one or more strain gauges can then be cross correlated by the processor algorithm with the data from the three dimensional motion sensor and or EMG data to provide a highly accurate representation of maternal contractions. Furthermore, over time the algorithm is trained with real world data to improve the analysis thereof. The main body 32 preferably further comprises a power supply such as a battery (not shown) or the like, although the main body 32 may of course be connected to an external power supply. It is also desirable that the main body 32 is relatively small in size and is comfortable to be worn for the extended periods of labour. While secured in position by the band 34 in the embodiment illustrated it is envisaged that the contraction sensor 12 could be provided as a patch form factor to be adhered in place for the duration of labour.

The contraction sensor 12 preferably additional comprises a separate sensor head 36 operable to monitor maternal heartrate, the sensor head 36 preferably being secured to the main body 32 via a flexible lead 38 although a wireless connection may be employed. The sensor head 36 may be secured to the maternal abdomen by any suitable means, and for example an adhesive backed film 40 may be provided on one face of the sensor head 36. The sensor head 36 may be adapted to measure heartrate by any suitable means but preferably utilises reflectance based pulse oximetry, employing an array of light emitters 42 such as LEDs or the like and a corresponding detector 44. It will however be appreciated that a convention fingertip worn heartrate sensor may be employed as part of the monitoring system of the invention to monitor the maternal heartrate, with data from the fingertip sensor being supplied to the processor. The power supply for the main body 32 is used to operate the emitters 42 and detector 44, in addition to any wireless data transmitter for sending the data to the processor (not shown). The maternal contractions and heartrate can therefore be continuously or intermittently monitored and the processor can be utilised to cross-correlate with the fetal heartrate to explain any changes in the fetal heartrate dynamics. For example it is normal for fetal heartrate to increase during maternal contractions and the algorithm may be operable to screen for these increases in order to eliminate such fetal heartrate increases from being alerted as potential fetal distress.

The monitoring system of the invention may also comprise a displacement sensor (not shown) operable to monitor the displacement of the probe relative to a reference location, preferably a fixed location on the mother, in order to monitor displacement of the fetus and thus the progression of labour. In a preferred arrangement the displacement sensor is operable to measure and monitor the distance between the probe 10 and the contraction sensor 12, although a separate sensor (not shown) may be provided such as a patch to be worn on the maternal abdomen or the like, in order to allow the distance between the sensor and probe 10 to be measured. The measurement of the distance between these two locations may be achieved by any suitable means, and could for example use any suitable form of wireless communication between the two locations to monitor and measure changes in the distance therebetween. The displacement sensor will therefore allow intra-partum measurement of the advancement of the fetal head as the probe 10 and contraction sensor 12 alter in distance from one another.

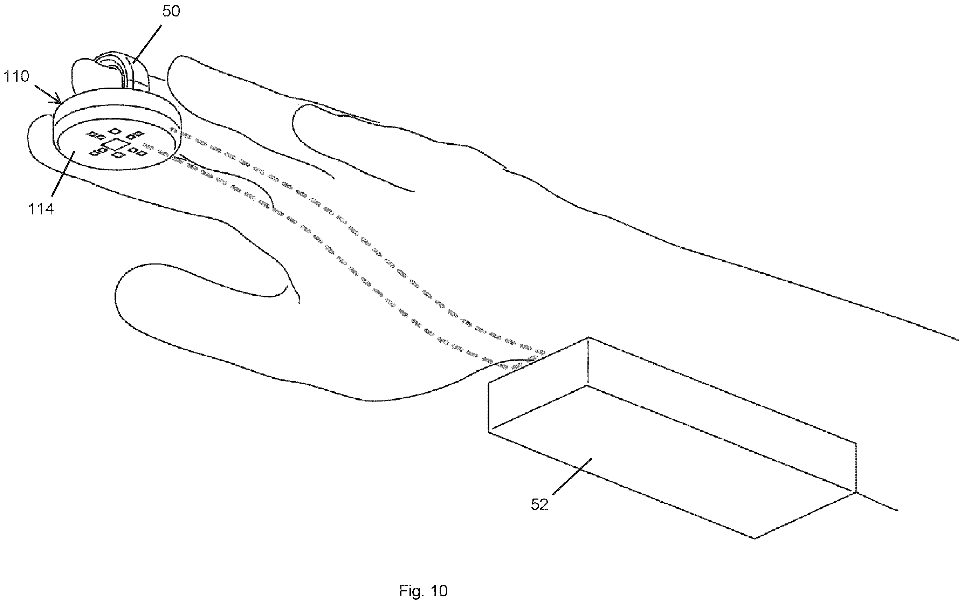

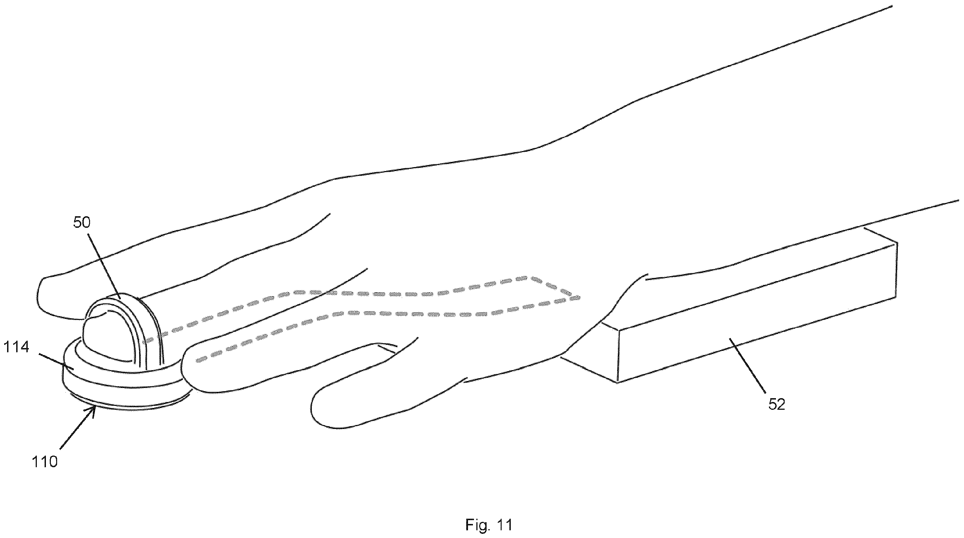

Referring now to Figures 10 and 11 there is illustrated an alternative embodiment of a fetal probe forming part of the monitoring system according to the present invention, generally indicated as 110. This alternative probe 110 is designed for intermittent contact with the fetal scalp in order to measure heartrate and optionally the additional bio-markers or parameters detailed above with respect to the probe 10 that is adhered to the scalp. The probe 110 thus comprises at least an optical sensor to measure heartrate, and optionally SpO₂, but may also include a temperature sensor, impedance sensor, etc. as hereinbefore described. The probe 110 thus operates in the same manner, but is configured to be secured to a fingertip of the clinician, for example by means of a simple ring or loop 50 provided on a housing 114 of the probe 110. In this way the clinician can apply the probe 110 against the fetal scalp during a digital examination, allowing the necessary heartrate and other measurements to be taken, and the probe 110 can then be removed until the next measurement is to be taken. The probe 110 does not therefore require any form of suction cup or vacuum generation as it is simply held in place on the scalp by the clinician.

The monitoring system optionally comprises a remote unit 52 which may be physically connected to the probe 110 and which may house a power supply for the probe 110, a processor (not shown), and a wireless transmitter to allow data to be sent from the unit 52 to a remote console (not shown) or other suitable location. The unit 52 may also be adapted to receive data from the contraction sensor 12 in order to cross correlated data with data from the fetus supplied by the probe 110. In the embodiment illustrated the remote unit 52 is arranged to be worn about the arm of the clinician but it will be appreciated that this is not a requirement.

Thus in use the monitoring system of the invention is operated to emit light onto the fetal scalp from the probe 10; 110 and the processor will then interpret the reflected light signal in order to calculate the heartrate, which can then be presented via any suitable output to the clinician. The reflected light may also be utilised to determine SPO₂ levels or other parameters with the use of a spectrometer to acquire spectral data for the reflected light. Preferably sequentially but potentially simultaneously the additional sensors (not shown) are used to measure temperature, hydration, EEG, etc. At the same time the contraction sensor 12 is monitoring contractions and providing data to the processor to be presented via suitable output to the clinician and optionally to be cross correlated with the fetal data by the processing algorithm. The contraction data is preferably sufficiently detailed to distinguish between pre-contraction, contraction and post contraction, but equally may simply be a square wave or binary signal denoting the presence or absence of a contraction. The maternal heartrate is also preferably fed to the processor for interpretation and cross correlation with the other data signals.

The data processing algorithm running on the processor will then apply the requisite background and/or reference measurements to the fetal and maternal data, for example factoring in the influence of the water spectra based on the water content of the irradiated tissue when measuring SPO₂ levels, which water content may for example be determined by the impedance sensor in the probe 10; 110. The tissue water content and absorption at the NIR wavelengths and a water spectrum may therefore be utilised by the algorithm as a reference spectrum along with the lipid spectrum and the standard background and correction for the emitters and detector. The data processing algorithm may also include correlated and auto correlated sampling techniques, machine learning and Al.

The algorithm may then examine the full spectral data and/or divide the data into certain spectral bands, and further process the data, for example using second order differentials, with Partial Least Squares Regression (PLS-R). The aim of PLS-R is to build a linear model of a set of response variables (Lactate or pH values) and the predictor matrix (reflectance spectra). PLS-R projects the data to a reduced dimension matrix space but also projects the data labels to this matrix space, allowing for a regression model to be built. PLS is a linear method that extracts a linear model from the data. Due to the high dimensionality of NIR spectra, PLS is the most widely used tool for modelling the spectral data. However, other algorithms for building non-linear models such as artificial neural networks (ANN) or support vector regression (SVR) may be applied as a part of the algorithmic processing of the raw data. A dimension reduction or feature selection method like PCA should be used before applying these algorithms.

The processed data may be utilised in a feedback loop to further refine the measurement parameters, wavelength ranges, etc. In addition the data may be further processed or combined with additional data obtained by the sensors of the monitoring system, for example temperature, impedance and pH data, etc. This additional data may be applied to further refine the data output from the algorithm, which can then be displayed in an appropriate format to the clinicians. For example the raw data, analysed data and/or alarms may be displayed in real time. Such alarms may be indicative of various bio-signs such as hypoxia.

The present invention thus provides a multimodal monitoring system which interprets data taken from the fetal scalp for the duration of labour along with contraction data. The fetal heartrate, the maternal heartrate and the contractions are all presented to the clinician on a continuous chart or time series. The clinician can then clearly identify and monitor the fetal heart rate as a priority looking for a low heartrate or an abnormally high heartrate and cross referencing to the maternal heartrate and contractions to ensure that there is a difference. Each of these signals can be separately and clearly displayed to the clinician to avoid any confusion or lack of specific data during labour. As noted, during contractions it is normal for the fetal and maternal heartrate to change due to trauma and this is allowed for by the clinician. Hence the requirement for contraction sensing to support heartrate monitoring. Principally the clinician is looking for atypical changes in fetal heartrate outside of contractions to predict fetal distress. The use of optical sensors in combination with contraction data and a novel processing algorithm to correlate and cross correlate the two or more data signals enables the monitoring system to extract, in real time, a clear fetal heartrate distinct from the maternal heartrate in addition to additional optional bio-markers such as pH and lactate signals from the light spectra. The monitoring system and method of the invention therefore allows for accurate measuring of fetal heartrate during labour to provide clinicians with a significantly improved, non-invasive means by which to monitor the fetus for intra-partum wellbeing against issues such as hypoxia.

The invention is not limited to the embodiments described herein but can be amended or modified without departing from the scope of the present invention.

## Claims

1. An intra-partum fetal monitoring system comprising a probe adapted for in-utero contact with vascularised tissue on the head of a fetus and operable to monitor fetal heartrate and/or fetal blood oxygen levels; a contraction sensor adapted to monitor maternal contractions; and a processor operable to convert real time data from the probe and real time data from the contraction sensor into correlated real time data regarding one or more biological markers to enable an assessment of intra-partum fetal wellbeing.

2. A fetal monitoring system according to claim 1 in which the probe comprises at least one optical sensor operable to emit light at a wavelength of between 500nm-950nm onto the vascularised tissue and to detect the reflected light.

3. A fetal monitoring system according to claim 2 in which the optical sensor comprises at least one multispectral light source.

4. A fetal monitoring system according to any preceding claim in which the contraction sensor comprises a three dimensional motion sensor and/or a vibration sensor and/or an electromyography sensor and/or a strain gauge.

5. A fetal monitoring system according to any preceding claim in which the contraction sensor comprises a heartrate sensor operable to measure maternal heartrate.

6. A fetal monitoring system according to claim 5 in which the heartrate sensor comprises at least one optical sensor operable to emit light onto maternal tissue and to detect the reflected light.

7. A fetal monitoring system according to any preceding claim in which the probe and/or the contraction sensor comprises a plurality of optical emitters and/or a plurality of optical detectors.

8. A fetal monitoring system according to claim 6 in which at least one of the optical detectors comprises a spectrometer.

9. A fetal monitoring system according to any preceding claim in which the probe and/or the contraction sensor comprises a temperature sensor and/or an impedametric sensor.

10. A fetal monitoring system according to any preceding claim in which the probe comprises a discrete pH sensor.

11. A fetal monitoring system according to any preceding claim in which the probe and/or the contraction sensor comprises a discrete power supply.

12. A fetal monitoring system according to any preceding claim in which the processor is operable to convert real time spectroscopy data from the probe into measurements of one or more of fetal blood lactate level, blood pH level, blood oxygen saturation, blood carbon dioxide level, body temperature, hydration and heart rate.

13. A fetal monitoring system according to any preceding claim in which the probe is operable to monitor oxygen saturation.

14. A fetal monitoring system according to any preceding claim in which the probe comprises two or more electrodes operable to convey an electrical signal through the vascularised tissue to enable measurement of hydration and/or pH.

15. A fetal monitoring system according to any preceding claim in which the probe and/or the contraction sensor comprises an electroencephalogram sensor.
